# EUROPÄISCHE PATENTANMELDUNG

(11) **EP 4 652 981 A1**
(43) Veröffentlichungstag der Anmeldung: **26.11.2025**
(21) Anmeldenummer: 24176989.2
(22) Anmeldetag: 21.05.2024
(51) Int. Cl.: A61K 6/62, A61K 6/887, B33Y 70/00, B33Y 70/10, B33Y 80/00

(54) **STRAHLUNGSHÄRTENDE ZUSAMMENSETZUNG FÜR DIE HERSTELLUNG DENTALER BAUTEILE**

(71) Anmelder: Kulzer GmbH, 63450 Hanau (DE)
(72) Erfinder: Barther, Dennis, 63500 Seligenstadt (DE); Meier, Christoph, 63486 Bruchköbel (DE)
(74) Vertreter: Pelster Behrends Patentanwälte PartG mbB

(57) **Zusammenfassung**

Die Erfindung betrifft ein eine strahlungshärtende Zusammensetzung für die Herstellung dentaler Bauteile im DLP-Verfahren oder SLA-Verfahren, umfassend bezogen auf die Gesamtmasse der strahlungshärtenden Zusammensetzung: i) ein oder mehrere radikalisch polymerisierbare Monomere in einem kombinierten Massenanteil von 60 % oder mehr, ii) eine oder mehrere Hexaarylbiimidazol-Verbindungen in einem kombinierten Massenanteil im Bereich von 0,1 bis 5 %, und iii) eine oder mehrere Mercaptotetrazol-Verbindungen in einem kombinierten Massenanteil im Bereich von 0,1 bis 5 %, wobei der kombinierte Massenanteil an Füllstoffen in der strahlungshärtenden Zusammensetzung weniger als 30 % beträgt.

## Beschreibung

Die Erfindung betrifft eine strahlungshärtende Zusammensetzung für die Herstellung dentaler Bauteile im DLP-Verfahren oder SLA-Verfahren, ein durch strahlungsinduzierte Polymerisation einer solchen strahlungshärtenden Zusammensetzung herstellbares dentales Bauteil und eine Verwendung eines spezifischen Initiatorsystems in einer strahlungshärtenden Zusammensetzung, zur Verbesserung der mechanischen Eigenschaften der im DLP-Verfahren oder SLA-Verfahren daraus herstellbaren dentalen Bauteile.

Der technologische Fortschritt und die fortschreitende Digitalisierung, die im letzten Jahrzehnt die Industrielandschaft verändert haben, beeinflussen auch den Bereich der Dentaltechnologie, wobei sie den Arbeitsalltag von Zahnärzten und Zahntechnikern grundlegend verändert haben. Über Jahrzehnte hinweg wurden viele Arten von Zahnersatz, wie beispielsweise Zahnkronen, Brücken, Teil- und Vollprothesen oder Inlays sowie kieferorthopädische Behandlungsgeräte, beispielsweise Schienen, vor allem manuell durch Zahntechniker hergestellt.

Der traditionelle Vorgang wird heutzutage vermehrt durch den Einsatz rechnergestützter Produktions- und Fertigungsverfahren unterstützt oder ersetzt, wobei diese Technologie vom Fachmann zuweilen auch als digitale Prothetik bezeichnet wird.

In den meisten Fällen sind dabei sogenannte CAD/CAM-Verfahren integraler Bestandteil der rechnergestützten Produktion. Der Ausdruck CAD bezeichnet das rechnergestützte Konstruieren ("computer-aided design"), welches im weiteren Sinne die Erzeugung eines digitalen Bauteils bezeichnet, das gegebenenfalls am Rechner direkt vom Anwender modifiziert werden kann. Der Ausdruck CAM bezeichnet die rechnerunterstützte Fertigung ("computer-aided manifacturing"), also die Umsetzung des mittels CAD erzeugten Bauteils in einen Code, der beispielsweise für die Steuerung spanabtragenden Maschine oder eines 3D-Druckers verwendet werden kann. Im Bereich der Dentaltechnik hat sich dabei für die Erstellung des CAD-Bauteils zunehmend der Einsatz sogenannter Intraoralscanner etabliert, die kontaktlos eine präzise Aufnahme des Mundraums des Patienten an den Rechner übermitteln können.

Für die Dentaltechnik von entscheidender Bedeutung sind insbesondere die additiven Fertigungsverfahren, bei denen Produkte aus formlosen Materialien ohne Einsatz spezieller Werkzeuge und auf Grundlage der durch CAD erzeugten Computerdatensätze hergestellt werden. Diese 3D-Druck-Verfahren, die zum Teil nach dem englischen Ausdruck auch als "rapid prototyping"-Verfahren bezeichnet werden, ersetzen oder ergänzen im Bereich der Zahntechnik heutzutage viele Arbeitsschritte bei der Herstellung von dentalen Bauteilen.

Eine prominente Rolle bei den dentalen 3D-Druck-Verfahren spielen die sogenannte Stereolithographie (SLA) und das Digital Light Processing (DLP). In diesen Verfahren wird eine strahlungshärtende Zusammensetzung vorgelegt und durch ortsaufgelöste, gezielte Bestrahlung an den gewünschten Stellen Schicht um Schicht ausgehärtet. Hierbei wird das entstehende Bauteil beispielsweise schrittweise in die Zusammensetzung abgesenkt oder schrittweise aus dieser herausgehoben, sodass nach jedem Inkrement über der zuletzt gebildeten Schicht jeweils nur ein dünner Film der strahlungshärtende Zusammensetzung vorliegt, der in etwa der Dicke der nächsten zu polymerisierenden Schicht entspricht. SLA- und DLP-Verfahren ähneln sich hinsichtlich ihres Grundprinzips, sind jedoch in der apparativen Ausgestaltung durchaus unterschiedlich, wobei beim SLA-Verfahren beispielsweise ein Laser zum Einsatz kommt, der die herzustellende Struktur nacheinander abläuft, wohingegen beim DLP-Verfahren durch eine geeignete Projektionstechnik die zeitgleiche Belichtung einer gesamten Fläche erfolgt. Das Prinzip der SLA- und DLP-Verfahren ist aus dem Stand der Technik bekannt und beispielsweise in der US 4575330 A oder der WO 2014078537 A1 offenbart. Der Einsatz von additiven Fertigungsverfahren in der Dentaltechnik ist beispielsweise aus den Dokumenten US 979554 B2, WO 2023126943 A2, DE 102016107935 A1 und DE 1020122011371 A1 bekannt und wird auch in der EP 3020361 B1 beschrieben.

Trotz der zunehmenden Verwendung entsprechender strahlungshärtender Zusammensetzungen besteht im Bereich der Technik ein fortgesetztes Interesse daran, die eingesetzten Materialien zu optimieren. Insbesondere wird angestrebt, die Verarbeitungseigenschaften der strahlungshärtenden Zusammensetzungen zu optimieren und gleichzeitig die Eigenschaften der daraus durch Aushärtung herstellbaren Materialien zu verbessern, insbesondere deren mechanische Eigenschaften.

Bei der Konzeption von strahlungshärtenden Zusammensetzungen für den Einsatz in 3D-Druck-Verfahren kommt dem Initiatorsystem regelmäßig eine entscheidende Bedeutung zu. Mit den eingesetzten Initiatoren müssen nicht nur gute mechanische Eigenschaften des Polymerisats gewährleistet werden, sondern es muss auch eine vorteilhafte Polymerisationskinetik realisiert werden. Neben der Polymerisationsgeschwindigkeit, welche durch die eingesetzten Initiatoren erzielbar ist, kommt dabei insbesondere auch der Frage der Polymerisationstiefe eine wichtige Bedeutung zu. Darüber hinaus ist von Bedeutung, welche initiale Strahlungsdosis benötigt wird, um die Polymerisationsreaktion zu initiieren und welche mittleren Schichtdicken mit einer vorgegebenen Strahlungsdosis erzielt werden können. Insbesondere die Tiefe der Polymerisation korreliert dabei teilweise auch mit der für die Initiierung verwendeten Wellenlänge der elektromagnetischen Strahlung. Diese Wellenlänge hat darüber hinaus jedoch auch weitere wichtige Implikationen für die eingesetzten 3D-Druck-Vorrichtungen. Die Effizienz einer strahlungshärtenden Zusammensetzung in 3D-Druck hängt maßgeblich davon ab, wie gut die eingesetzten 3D-Druck-Vorrichtungen die optimalen Wellenlängen des Initiatorsystems bereitstellen können.

Im Lichte des vorstehend beschriebenen Spannungsfelds aus unterschiedlichen Anforderungen wurden in der Vergangenheit zahlreiche vorteilhafte Initiatorsysteme identifiziert.

Hierbei sind beispielsweise Phosphinoxid-basierte sogenannte Norrish Typ I Initiatoren, wie beispielsweise Phenylbis(2,4,6-trimethylbenzoyl) phosphinoxid (BAPO), Diphenyl(2,4,6-trimethylbenzoyl)phosphinoxid (TPO) und Ethyl(2,4,6-trimethylbenzoyl) phenylphosphinat (TPO-L), aufgrund des breiten Absorptionsspektrums weit verbreitet. Verglichen mit sogenannten Norrish Typ II Initiatoren, wie beispielsweise dem ebenfalls etablierten Campherchinon/Amin-System, ist das Maximum des Absorptionsspektrums jedoch zumeist im kurzwelligeren UV-Bereich lokalisiert und ermöglicht daher lediglich geringe Aushärtetiefen und eingeschränkte Nutzbarkeit auf einigen 3D-Druckern. Eine hohe Aushärtetiefe ist speziell bei der Nachbelichtung ("Post Curing") vorteilhaft und erzeugt bessere mechanische Eigenschaften der gedruckten Materialien.

Ein weiterer Nachteil bei der Verwendung von Norrish Typ I Initiatoren besteht darin, dass die Polymerisationsreaktivität des zu verdruckenden Materials nur schlecht kontrolliert werden kann, da es sich um ein Ein-Komponenten-Initiatorsystem handelt.

Die Wahl geeigneter Initiatoren wird dabei im Zuge eines steigenden Bewusstseins für Umwelt- und/oder Gesundheitsaspekte zunehmend dadurch erschwert, dass viele der aus dem Stand der Technik bekannten Initiatorsysteme unter Umwelt- und/oder Gesundheitsgesichtspunkten heute kritisch gesehen werden und ihr Einsatz in einigen Ländern bereits eingeschränkt ist oder eine entsprechende zeitnahe Einschränkung nicht unwahrscheinlich ist. Trotz ihrer guten Performance im Sinne mechanischer Eigenschaften und Reaktivität sowie deren vielseitige Verwendung stehen Phosphinoxid-basierte Initiatoren im Verdacht reproduktionstoxische Auswirkungen auf den menschlichen Körper zu haben. TPO wurde beispielsweise bereits in die Liste der "Substances of very high concern" der Europäischen Chemikalienagentur (ECHA) aufgenommen und als potentiell kanzerogen, mutagen und reproduktionstoxisch (CMR) klassifiziert (Klasse 2), was den generellen Einsatz dieser Stoffklasse in Medizinprodukten in Frage stellt.

Die primäre Aufgabe der vorliegenden Erfindung war es, die Nachteile des Standes der Technik auszuräumen oder zumindest abzuschwächen.

Insbesondere war es die Aufgabe der vorliegenden Erfindung, eine strahlungshärtende Zusammensetzung anzugeben, deren Initiatorsystem unter Umwelt- und/oder Gesundheitsgesichtspunkten weniger problematisch ist als die aus dem Stand der Technik bekannten Lösungen, wobei es eine wünschenswerte Vorgabe war, dass das Initiatorsystem möglichst weltweit keinen regulatorischen Beschränkungen unterliegen sollte, insbesondere im Zusammenhang mit der additiven Fertigung von Medizinprodukten.

Hierbei war es eine wichtige Aufgabe der vorliegenden Erfindung, dass die anzugebende strahlungshärtende Zusammensetzung über eine vorteilhafte Polymerisationskinetik verfügen sollte, insbesondere hinsichtlich der für die Initiierung benötigten Strahlungsdosis sowie hinsichtlich der mittleren Schichtdicke, welche bei einer gegebenen Strahlungsdosis erreicht werden kann.

Es war eine weitere Aufgabe der vorliegenden Erfindung, dass die anzugebende strahlungshärtende Zusammensetzung in vorteilhafte Polymerisate überführbar sein sollte, welche vorteilhafte mechanische Eigenschaften aufweisen, die diese auch für Hochleistungsanwendungen im dentalen Bereich qualifizieren.

Insoweit war es eine Aufgabe der vorliegenden Erfindung, dass die anzugebende strahlungshärtende Zusammensetzung hinsichtlich der Polymerisationskinetik sowie der mechanischen Eigenschaften des Polymerisates mit aus dem Stand der Technik bekannten, strahlungshärtenden Zusammensetzungen, welche etablierte Initiatorsysteme verwenden, insbesondere solche vom Norrisch Typ I, möglichst dahingehend vergleichbar sein sollte, dass diese Eigenschaften zumindest auf einem vergleichbaren Niveau liegen, wobei es wünschenswert war, dass diese Eigenschaften zumindest teilweise sogar verbessert werden, sodass die diesbezüglich bestehenden Zielkonflikte besser gelöst werden.

Es war eine wünschenswerte Vorgabe der vorliegenden Erfindung, dass die anzugebenden strahlungshärtenden Zusammensetzungen insbesondere auch effizient in modernen 3D-Druckern verarbeitbar sein sollten, d. h. dass eine Aushärtung bei Wellenlängen von 385 nm oder weniger effizient möglich sein sollte, wobei es eine wünschenswerte Eigenschaft war, dass die anzugehenden Initiatorsysteme einen hinreichend breiten Anregungsbereich aufweisen, dass diese mit einer breiten Spannbreite an bestehenden 3D-Druckern, insbesondere auch solchen, welche mit Wellenlängen von etwa 405 nm operieren, ausgehärtet werden können.

Es war eine ergänzende Aufgabe der vorliegenden Erfindung, ein dentales Bauteil bereitzustellen, welches aus den anzugebenden strahlungshärtenden Zusammensetzungen herstellbar ist und welches nicht nur in besonders zeit- und kosteneffizienter Art und Weise gefertigt werden kann, sondern welches darüber hinaus auch über ausgezeichnete mechanische Eigenschaften verfügt und zudem hinsichtlich umwelt- und gesundheitlichen Aspekten als vorteilhaft bewertet wird.

Es war eine weitere Aufgabe der vorliegenden Erfindung, darüber hinaus die Verwendung eines spezifischen Initiatorsystems zur Verbesserung der mechanischen Eigenschaften von im 3D-Druck hergestellten dentalen Bauteilen anzugeben.

Die Erfinder der vorliegenden Erfindung haben nunmehr gefunden, dass sich die vorstehend beschriebenen Aufgaben überraschenderweise lösen lassen, wenn in einer strahlungshärtenden Zusammensetzung, welche über einen hohen Anteil an radikalisch polymerisierbaren Monomeren und einen vergleichsweise geringen Anteil an Füllstoffen verfügt, ein Initiatorsystem eingesetzt wird, welches Hexaarylbiimidazol-Verbindungen in Verbindung mit Mercaptotetrazol-Verbindungen einsetzt, wie es in den Ansprüchen definiert ist.

Überraschenderweise wurde für den Einsatz eines entsprechenden Initiatorsystems in den spezifischen füllstoffarmen, strahlungshärtenden Zusammensetzungen eine vorteilhafte Aushärtekinetik, insbesondere hinsichtlich der initial benötigten Strahlungsdosis, gefunden, wobei darüber hinaus vorteilhafte mechanische Eigenschaften der erhaltenen Polymerisate festgestellt wurden. In der Kombination mit den weiteren Bestandteilen der strahlungshärtenden Zusammensetzung erlaubt die Verwendung des Initiatorsystems dabei in der Gesamtheit Leistungseigenschaften, die mit denen aus dem Stand der Technik bekannten, etablierten Initiatorsystemen zumindest vergleichbar und teilweise sogar verbessert sind. Die Kombination von Hexaarylbiimidazol-Verbindungen mit Mercaptotetrazol-Verbindungen ist dabei unter Umwelt- und Gesundheitsgesichtspunkten gegenüber vielen der aus dem Stand der Technik bekannten Initiatorsysteme bevorzugt, sodass das identifizierte Initiatorsystem für den Einsatz bei der additiven Fertigung von Medizinprodukten besonders vorteilhaft ist.

Die vorstehend genannten Aufgaben werden somit durch den Gegenstand der Erfindung gelöst, wie er in den Ansprüchen definiert ist. Bevorzugte erfindungsgemäße Ausgestaltungen ergeben sich aus den Unteransprüchen und den nachfolgenden Ausführungen.

Solche Ausführungsformen, die nachfolgend als bevorzugt bezeichnet sind, werden in besonders bevorzugten Ausführungsformen mit Merkmalen anderer als bevorzugt bezeichneter Ausführungsformen kombiniert. Ganz besonders bevorzugt sind somit Kombinationen von zwei oder mehr der nachfolgend als besonders bevorzugt bezeichneten Ausführungsformen. Ebenfalls bevorzugt sind Ausführungsformen, in denen ein in irgendeinem Ausmaß als bevorzugt bezeichnetes Merkmal einer Ausführungsform mit einem oder mehreren weiteren Merkmalen anderer Ausführungsformen kombiniert wird, die in irgendeinem Ausmaß als bevorzugt bezeichnet werden. Merkmale bevorzugter dentaler Bauteile und Verwendungen ergeben sich aus den Merkmalen bevorzugter strahlungshärtender Zusammensetzungen.

Insoweit nachfolgend für ein Element, beispielsweise für die radikalisch polymerisierbare Monomere oder die Füllstoffe, sowohl spezifische Mengen bzw. Anteile dieses Elementes als auch bevorzugte Ausgestaltungen des Elementes offenbart werden, sind insbesondere auch die spezifischen Mengen bzw. Anteile der bevorzugt ausgestalteten Elemente offenbart. Zudem ist offenbart, dass bei den entsprechenden spezifischen Gesamtmengen bzw. Gesamtanteilen der Elemente zumindest ein Teil der Elemente bevorzugt ausgestaltet sein kann und insbesondere auch, dass bevorzugt ausgestaltete Elemente innerhalb der spezifischen Gesamtmengen oder Gesamtanteile wiederum in den spezifischen Mengen bzw. Anteilen vorliegen können.

Insbesondere bevorzugte Ausführungsformen der Erfindung sind in den Ausführungsbeispielen offenbart. Besonders bevorzugte Ausführungsformen der Erfindung weisen vor diesem Hintergrund zwei oder mehr, bevorzugt drei oder mehr, ganz besonders bevorzugt vier oder mehr, der nachfolgend offenbarten bevorzugten Merkmale der Erfindung auf, welche auch in den Ausführungsbeispielen realisiert sind.

Die Erfindung betrifft insbesondere eine strahlungshärtende Zusammensetzung für die Herstellung dentaler Bauteile im DLP-Verfahren oder SLA-Verfahren, umfassend bezogen auf die Gesamtmasse der strahlungshärtenden Zusammensetzung:
i) ein oder mehrere radikalisch polymerisierbare Monomere in einem kombinierten Massenanteil von 60 % oder mehr,
ii) eine oder mehrere Hexaarylbiimidazol-Verbindungen in einem kombinierten Massenanteil im Bereich von 0,1 bis 5 %, und
iii) eine oder mehrere Mercaptotetrazol-Verbindungen in einem kombinierten Massenanteil im Bereich von 0,1 bis 5 %,
wobei der kombinierte Massenanteil an Füllstoffen in der strahlungshärtenden Zusammensetzung weniger als 30 % beträgt.

Die erfindungsgemäße strahlungshärtende Zusammensetzung ist für die Herstellung dentaler Bauteile in einem 3D-Druck-Verfahren, nämlich einem DLP-Verfahren oder SLA-Verfahren geeignet und bestimmt. Strahlungshärtende Zusammensetzungen für diesen Zweck sind im Allgemeinen ebenso bekannt wie die für das DLP-Verfahren oder SLA-Verfahren nutzbaren Vorrichtungen, wobei entsprechende Produkte und Vorrichtungen von verschiedenen Anbietern kommerziell erhältlich sind, beispielsweise von der Firma Kulzer.

Die Ausdrücke "DLP-Verfahren" und "SLA-Verfahren" bezeichnen die vorstehend beschriebenen Verfahren des Digital Light Processings bzw. der Stereolithographie, die dem Fachmann als Methoden bekannt sind. Die Geeignetheitsangabe der strahlungshärtenden Zusammensetzung für die Herstellung dentaler Bauteile in diesen Verfahren stellt insbesondere eine funktionale Anforderung an die Viskosität des Materials, insoweit, dass diese hinreichend niedrig sein sollte. Hierdurch werden viele strahlungshärtende Zusammensetzungen, die an anderer Stelle im Dentalbereich eingesetzt werden, insbesondere solche mit hohen Füllstoffgehalten, beispielsweise keramische Schlicker, ausgeschlossen. Bevorzugt ist hinsichtlich der Flüssigkeitseigenschaft eine erfindungsgemäße strahlungshärtende Zusammensetzung, wobei die strahlungshärtende Zusammensetzung bei 23 °C eine dynamische Viskosität im Bereich von 0,001 bis 10 Pa s, bevorzugt im Bereich von 0,5 bis 5 Pa s, besonders bevorzugt im Bereich von 0,7 bis 2,5 Pa s, aufweist. Die Viskosität der nichtausgehärteten strahlungshärtenden Zusammensetzungen wird im Rahmen der vorliegenden Erfindung mit Hilfe eines Rheometers Anton Paar - Physica MCR301 bei 23 °C bestimmt (Wechselplatte I-PP-50/SS glatt und Messkegel CP25-1, 25 mm, 1°). Die Spaltbreite ist gegeben durch den Winkel und Durchmesser des Kegels. Die Messung wird bei konstanter Rotation und einer Scherrate von 100 1/s durchgeführt. Insgesamt werden 10 Messpunkte aufgezeichnet. Eine Messpunktdauer beträgt 6 s bei einer Abschnittszeit von 60 s.

Die vorstehende Geeignetheit schließt dabei jedoch nicht aus, dass die erfindungsgemäßen strahlungshärtenden Zusammensetzungen auch in anderen additiven Fertigungsverfahren zum Einsatz kommen können, beispielsweise im Rahmen der sogenannten "Ink Jet" Technologie. Nach Einschätzung der Erfinder werden die erfindungsgemäßen strahlungshärtenden Zusammensetzungen in der späteren Praxis jedoch vor allem im DLP-Verfahren eingesetzt werden. Bevorzugt ist entsprechend eine erfindungsgemäße strahlungshärtende Zusammensetzung, wobei die strahlungshärtende Zusammensetzung für die Herstellung dentaler Bauteile im DLP-Verfahren geeignet ist.

Im Rahmen der vorliegenden Erfindung werden die definierten Bestandteile der strahlungshärtenden Zusammensetzung in Übereinstimmung mit dem fachmännischen Verständnis jeweils als "ein oder mehrere" eingesetzt. Die Bezeichnung "ein oder mehrere" bezieht sich dabei in branchenüblicher Weise auf die chemische Natur der entsprechenden Verbindungen und nicht auf deren Stoffmenge.

Insoweit im Rahmen der Erfindung Massenanteile angegeben werden, werden diese in branchenüblicher Weise jeweils als kombinierte Massenanteile der einen oder der mehreren Komponenten angegeben, wodurch ausgedrückt wird, dass der Massenanteil der entsprechend ausgebildeten Komponenten zusammengenommen die entsprechenden Kriterien erfüllt.

Soweit nicht im Einzelfall etwas anderes definiert wird, beziehen sich die im Rahmen der vorliegenden Erfindung definierten Massenanteile für die Komponenten der strahlungshärtenden Zusammensetzung jeweils auf die Gesamtmasse der strahlungshärtenden Zusammensetzung. Der Fachmann versteht dabei, dass die Massenanteile mit der Maßgabe definiert werden, dass die Gesamtmassenanteile der strahlungshärtenden Zusammensetzung sich zu 100 % addieren, so dass definierte Obergrenze eines Bestandteils im Bedarfsfall anzupassen sind, so dass sie zusammen mit den Untergrenzen der weiteren verpflichtenden Bestandteile 100 % ergeben.

Der Ausdruck dentale Bauteile bezeichnet im Sinne der vorliegenden Erfindung prinzipiell zunächst sämtliche Bauteile und dreidimensionale Strukturen, die im Bereich der Zahntechnik als Hilfsmittel, Zwischenstufe oder Endprodukt angefertigt werden, d.h. unabhängig von der zugrundeliegenden dentalen Indikation. Wegen der vorteilhaften mechanischen Eigenschaften der durch Aushärtung der strahlungshärtenden Zusammensetzungen herstellbaren dentalen Bauteile ist die vorliegende Erfindung aber insbesondere bei der Herstellung von dentalen Bauteilen für permanente dentale Anwendungen, d.h. im Mund des Patienten geeignet. Beispielhaft ist demnach eine erfindungsgemäße strahlungshärtende Zusammensetzung, wobei die dentalen Bauteile ausgewählt sind aus der Gruppe bestehend aus dentalen Prothesen, dentalen Modellen, Gingiva-Masken, Aufbissschienen, CAD-to-cast-Gussformen, Abformlöffel, Bohrschablonen, temporären und permanenten Kronen, Alignern, Brücken, Onlays, Inlays und Veneers. Bevorzugt ist jedoch eine erfindungsgemäße strahlungshärtende Zusammensetzung, wobei die dentalen Bauteile ausgewählt sind aus der Gruppe bestehend aus dentalen Prothesen, temporären und permanenten Kronen, Alignern, Brücken, Onlays, Inlays und Veneers.

Der Ausdruck "strahlungshärtend", der vorstehend zur Charakterisierung der Zusammensetzung verwendet wird, entspricht dem in der Branche üblichen Fachterminus, wobei alternativ auch gleichwertig Begriffe wie "strahlungshärtbar", "strahlungsvernetzend" oder ähnliche Ausdrücke verwendet werden. Der Ausdruck ist dabei zu verstehen als die Eigenschaft der Zusammensetzung durch die Applikation von elektromagnetischer Strahlung, insbesondere im Bereich des ultravioletten bzw. sichtbaren Lichts, auszuhärten, indem durch die Strahlung mittels eines Initiators die Polymerisation von radikalisch polymerisierbaren Monomeren in der Zusammensetzung induziert wird. Die Wellenlänge der hierfür verwendeten Strahlung liegt zumeist im Bereich des sichtbaren Lichtes bzw. der angrenzenden Wellenlängenbereiche im UV-Bereich, wobei besonders häufig Wellenlängen im blauen und ultravioletten Bereich zum Einsatz kommen. In Übereinstimmung mit dem fachmännischen Verständnis wird die Strahlungshärtbarkeit durch die vorstehend definierte Kombination von radikalisch polymerisierbaren Monomeren mit Initiatoren erreicht. Bevorzugt ist dabei im Lichte der spezifischen Initiatoren eine erfindungsgemäße strahlungshärtende Zusammensetzung, wobei die strahlungshärtende Zusammensetzung dazu ausgelegt ist, bei Bestrahlung der strahlungshärtenden Zusammensetzung mit elektromagnetischer Strahlung im Wellenlängenbereich von 200 und 420 nm, bevorzugt im Wellenlängenbereich von 250 bis 415 nm, besonders bevorzugt im Wellenlängenbereich von 300 bis 410 nm, auszuhärten. Bevorzugt ist mit anderen Worten eine erfindungsgemäße strahlungshärtende Zusammensetzung, wobei das Photoinitatorsystem aus den Hexaarylbiimidazol-Verbindungen und den Mercaptotetrazol-Verbindungen dazu ausgelegt ist, bei Bestrahlung der strahlungshärtenden Zusammensetzung mit elektromagnetischer Strahlung im Wellenlängenbereich von 200 und 420 nm, bevorzugt im Wellenlängenbereich von 250 bis 415 nm, besonders bevorzugt im Wellenlängenbereich von 300 bis 410 nm, eine Polymerisation der polymerisierbaren Monomere zu initiieren.

Neben den nachfolgend weiter diskutierten Initiatoren bilden die erfindungsgemäß einzusetzenden radikalisch polymerisierbare Monomere einen wichtigen Bestanteil, welcher die Strahlungshärtbarkeit bedingt. Die radikalisch polymerisierbaren Monomere sind durch ihre Eigenschaft charakterisiert, nach Initiation durch einen Radikalstarter in einer Kettenreaktion miteinander zu vernetzen, wobei der Radikalstarter in strahlungshärtenden Zusammensetzungen regelmäßig durch den oder die Initiator(en) bereitgestellt wird. Vom Grundprinzip ist die vorliegende Erfindung nicht auf bestimmte radikalisch polymerisierbare Monomere beschränkt, sondern für alle radikalisch polymerisierbaren Monomere anwendbar, wobei diese zumeist eine endständige ungesättigte Doppelbindung aufweisen, über die die radikalische Polymerisation verlaufen kann. Diese Monomere, die monofunktionell oder multifunktionell sein können, wählt der Fachmann prinzipiell basierend auf den für das herzustellende dentale Bauteil gewünschten physikalisch-chemischen und anwendungstechnischen Eigenschaften aus. Im Bereich der Dentalchemie sind insbesondere (Meth)acrylate als Monomere von herausragender Bedeutung, wobei der Ausdruck (Meth)acrylate in Übereinstimmung mit dem Verständnis des Fachmanns sowohl Acrylate als auch Methacrylate bezeichnet. Diese im Bereich der Dentalchemie häufig eingesetzten Monomere sind dem Fachmann bekannt und beispielsweise in der EP 3020361 B1 oder der DE 3941629 C1 offenbart. Beispielhaft ist vor diesem Hintergrund eine erfindungsgemäße strahlungshärtende Zusammensetzung, wobei das eine oder die mehreren radikalisch polymerisierbaren Monomere ausgewählt sind aus der Gruppe bestehend aus (Meth)acrylsäure, (Meth)acrylaten, (Meth)acrylamiden und anderen Vinyl-Verbindungen, bevorzugt ausgewählt sind aus der Gruppe bestehend aus (Meth)acrylsäure, (Meth)acrylaten und (Meth)acrylamiden, besonders bevorzugt ausgewählt sind aus der Gruppe bestehend aus (Meth)acrylaten. Bevorzugt ist dabei eine erfindungsgemäße strahlungshärtende Zusammensetzung, wobei das eine oder die mehreren radikalisch polymerisierbaren Monomere ausgewählt sind aus der Gruppe bestehend aus monofunktionellen (Meth)acrylaten und polyfunktionellen (Meth)acrylaten.

Beispiele für geeignete monofunktionelle (Meth)acrylate sind beispielsweise ausgewählt aus der Gruppe bestehend aus substituierten und unsubstituierten Alkyl(meth)acrylaten und substituierten und unsubstituierten Cycloalkyl(meth)acrylaten, bevorzugt ausgewählt aus der Gruppe bestehend aus Dicyclopentanylmethylacrylat, Tertiobutylcyclohexl(meth)acrylat, 2(2-Ethoxyethoxy)ethyl(meth)acrylat, Tetrahydrofurfuryl(meth)acrylat, Octyldecyl(meth)acrylat, Isobornyl(meth)acrylat, Isodecyl(meth)acrylat und Alkyl(meth)acrylaten mit bis zu 12 Kohlenstoffatomen im Alkylrest.

Beispiele für geeignete polyfunktionellen (Meth)acrylate sind beispielsweise ausgewählt aus der Gruppe bestehend aus Urethandimethacrylat, BisGMA, Triethylenglycoldi(meth)acrylate (TEGD[M]A), Tricyclodecandimethanoldi(meth)acrylat (TCDDMD[M]A), Bisphenol A-ethoxylat (mit versch. Kettenlängen) (Bis-EMA), 1,6-Hexandioldi(meth)acrylat, Polyethylenglycoldi(meth)acrylat, 3-Methyl-1,5-pentandioldi(meth)acrylat, Dipropylenglycoldi-(meth)acrylat, Esterdioldi(meth)acrylat, Trimethylolpropantri(meth)acrylat) (TMPT[M]A), Tris(2-hydroxyethyl)isocyanurat-tri(meth)acrylat, Di-Pentaerythritolpenta(meth)acrylat), ethoxyliertes oder propoxyliertes Trimethylolpropantr(meth)acrylat, 2-Propenoic acid, 1,1'-[((octahydro-4,7,methano-1H-indene-5,diyl)bis(methyleneiminocarbonyloxy-2,1-ethanediyl)] ester (TCD-Di-HEA;CAS 861437-11-8), 2-propeonic acid,1,1'[(octahydro-4,7-methano-1H-indene-5-diyl)bis(methyleneoxycarbonylamino-2,1-ethanediyl)] ester (TCD-Di-UEA; CAS 945656-78-0), decanediol-1,10-dimethacrylate und Urethandimethacrylat sowie weiteren Urethan(meth)acrylaten und Thiourethan(meth)acrylaten, wie sie beispielsweise in der WO 2022248546 A1 offenbart werden.

Nach Einschätzung der Erfinder lassen sich dabei insbesondere durch den Einsatz bzw. die Kombination von mono- und difunktionellen (Meth)acrylaten besonders leistungsfähige strahlungshärtende Zusammensetzungen erhalten. Bevorzugt ist entsprechend eine erfindungsgemäße strahlungshärtende Zusammensetzung, wobei die strahlungshärtende Zusammensetzung zumindest ein radikalisch polymerisierbares Monomers umfasst, das ausgewählt ist aus der Gruppe bestehend aus monofunktionellen (Meth)acrylaten, und/oder wobei die strahlungshärtende Zusammensetzung zumindest ein radikalisch polymerisierbares Monomers umfasst, das ausgewählt ist aus der Gruppe bestehend aus polyfunktionellen (Meth)acrylaten, bevorzugt difunktionellen (Meth)acrylaten. In den Experimenten der Erfinder haben sich in Kombination mit dem erfindungsgemäß einzusetzenden Initiatorsystem insbesondere dann besonders vorteilhafte Eigenschaftsprofile ergeben, wenn in der Monomerkomponente sowohl monofunktionelle als auch difunktionelle Monomere eingesetzt werden.

Besonders bevorzugt ist hinsichtlich der Wahl der Monomere eine erfindungsgemäße strahlungshärtende Zusammensetzung, wobei die strahlungshärtende Zusammensetzung zumindest ein radikalisch polymerisierbares Monomers umfasst, das ausgewählt ist aus der Gruppe bestehend aus ethoxylierten 2 Bisphenol-A-dimethacrylaten, Tricyclodecanmethanolacrylaten, Urethandimethacrylaten und Carboxyfunktionalisierten Polyester Acrylaten, wobei die strahlungshärtende Zusammensetzung bevorzugt zwei oder mehr, besonders bevorzugt drei oder mehr, insbesondere vier oder mehr, verschiedene dieser radikalisch polymerisierbaren Monomere umfasst.

Bevorzugt ist hinsichtlich des grundsätzlichen Gehaltes der Monomerkomponente eine erfindungsgemäße strahlungshärtende Zusammensetzung, wobei die strahlungshärtende Zusammensetzung das eine oder die mehreren radikalisch polymerisierbaren Monomere in einem kombinierten Massenanteil von 65 % oder mehr, bevorzugt 70 % oder mehr, besonders bevorzugt 75 % oder mehr, ganz besonders bevorzugt 85 % oder mehr, umfasst. Bevorzugt ist zusätzlich oder alternativ auch eine erfindungsgemäße strahlungshärtende Zusammensetzung, wobei die strahlungshärtende Zusammensetzung das eine oder die mehreren radikalisch polymerisierbaren Monomere in einem kombinierten Massenanteil im Bereich von 60 bis 96 %, bevorzugt im Bereich von 70 bis 93 %, besonders bevorzugt im Bereich von 80 bis 90 %, umfasst.

Ein wesentlicher Aspekt der erfindungsgemäßen strahlungshärtenden Zusammensetzung ist es, dass der kombinierte Massenanteil an Füllstoffen relativ niedrig gewählt wird, wobei die Erfinder es für bevorzugt erachten, tendenziell noch niedrigere Füllstoffgehalte einzustellen. Hierbei ist es denkbar und für einige Anwendungen auch bevorzugt, wenn die erfindungsgemäße strahlungshärtende Zusammensetzung gänzlich füllstofffrei ausgeführt wird. Bevorzugt ist somit eine erfindungsgemäße strahlungshärtende Zusammensetzung, wobei der kombinierte Massenanteil an Füllstoffen in der strahlungshärtenden Zusammensetzung 25 % oder weniger, bevorzugt 20 % oder weniger, besonders bevorzugt 15 % oder weniger, beträgt.

Auch wenn es, wie vorstehend erläutert, denkbar ist, die erfindungsgemäße strahlungshärtende Zusammensetzung füllstofffrei auszuführen, erachten es die Erfinder mit Blick auf die Realisierung von vorteilhaften mechanischen Eigenschaften als besonders bevorzugt, wenn der strahlungshärtenden Zusammensetzung bewusst ein Füllstoff zugesetzt wird. Bevorzugt ist entsprechend eine erfindungsgemäße strahlungshärtende Zusammensetzung, wobei die strahlungshärtende Zusammensetzung zusätzlich umfasst:
iv) einen oder mehrere Füllstoffe in einem kombinierten Massenanteil im Bereich von 0,05 bis 29 %.

Den Erfindern ist es in eigenen Versuchen gelungen, insoweit besonders geeignete Massenanteile für den eingesetzten Füllstoff anzugeben, mit denen sich im Zusammenspiel mit den erfindungsgemäß einzusetzenden Initiatorsystemen eine vorteilhafte Polymerisationskinetik mit ausgezeichneten mechanischen Eigenschaften erhalten lässt. Bevorzugt ist nämlich eine erfindungsgemäße strahlungshärtende Zusammensetzung, wobei die strahlungshärtende Zusammensetzung den einen oder die mehreren Füllstoffe in einem kombinierten Massenanteil von weniger als 25 %, bevorzugt weniger als 20 %, besonders bevorzugt weniger als 15 %, umfasst. Bevorzugt ist zusätzlich oder alternativ auch eine erfindungsgemäße strahlungshärtende Zusammensetzung, wobei die strahlungshärtende Zusammensetzung den einen oder die mehreren Füllstoffe in einem kombinierten Massenanteil im Bereich von 0,1 bis 25 %, bevorzugt im Bereich von 0,5 bis 22,5 %, besonders bevorzugt im Bereich von 1 bis 20 %, ganz besonders bevorzugt im Bereich von 2 bis 17,5 %, insbesondere bevorzugt im Bereich von 5 bis 15 % umfasst.

Nach Einschätzung der Erfinder kann für die Umsetzung der vorliegenden Erfindung prinzipiell auf solche Füllstoffe zurückgegriffen werden, welche im dentalen Bereich für vergleichbare Produkte häufig zum Einsatz kommen, bspw. Dentalgläser. Allerdings hat sich in den Experimenten der Erfinder gezeigt, dass sich insbesondere mit Füllstoffen aus Siliziumdioxid und Zirkondioxid, darunter vor allen Dingen mit dem auch als "gefällte Kieselsäure" bekannten Füllstoff, besonders vorteilhafte Eigenschaften erzielen lassen. Bevorzugt ist folglich eine erfindungsgemäße strahlungshärtende Zusammensetzung, wobei der eine oder die mehreren Füllstoffe ausgewählt sind aus der Gruppe bestehend aus oxidischen Füllstoffen, bevorzugt ausgewählt sind aus der Gruppe bestehend aus Siliciumdioxid und Zirkondioxid, besonders bevorzugt ausgewählt sind aus der Gruppe bestehend aus amorphem Siliciumdioxid, insbesondere ausgewählt sind aus der Gruppe bestehend aus gefälltem amorphem Siliciumdioxid. Bevorzugt ist dabei für im Wesentlichen alle Ausführungsformen eine erfindungsgemäße strahlungshärtende Zusammensetzung, wobei der eine oder die mehreren Füllstoffe ausgewählt sind aus der Gruppe bestehend aus partikulären Füllstoffen.

Ein wesentlicher Aspekt der vorliegenden Erfindung ist das Zusammenspiel von zwei spezifischen Verbindungsklassen im Initiatorsystem der erfindungsgemäßen strahlungshärtenden Zusammensetzung. Hierfür werden Hexaarylbiimidazol-Verbindungen mit Mercaptotetrazol-Verbindungen kombiniert, welche gemeinsam als Fotoinitiator bzw. als Co-Initiator fungieren und die Strahlungshärtbarkeit der erfindungsgemäßen strahlungshärtenden Zusammensetzungen bedingen.

In umfassenden eigenen Experimenten ist es den Erfindern gelungen, für die Gehalte der erfindungsgemäß einzusetzenden Bestandteile des Initiatorsystems bevorzugte Bereiche zu identifizieren, mit denen sich beim Einsatz im DLP- oder SLA-Verfahren jeweils ausgezeichnete Ergebnisse erzielen lassen. Im Zuge dieser Studien ist dabei identifiziert worden, dass es besonders vorteilhaft ist, die beiden Verbindungen zumindest im gleichen Massenanteil einzusetzen, wobei bevorzugt jedoch die Mercaptotetrazol-Verbindungen im Überschuss zugegeben werden. Bevorzugt ist hierbei zunächst eine erfindungsgemäße strahlungshärtende Zusammensetzung, wobei die strahlungshärtende Zusammensetzung die eine oder die mehreren Hexaarylbiimidazol-Verbindungen in einem kombinierten Massenanteil im Bereich von 0,15 bis 4,5 %, bevorzugt im Bereich von 0,2 bis 4,0 %, besonders bevorzugt im Bereich von 0,25 bis 3,5 %, ganz besonders bevorzugt im Bereich von 0,3 bis 3,0 % umfasst. Bevorzugt ist zusätzlich oder alternativ auch eine erfindungsgemäße strahlungshärtende Zusammensetzung, wobei die strahlungshärtende Zusammensetzung die eine oder die mehreren Mercaptotetrazol-Verbindungen in einem kombinierten Massenanteil im Bereich von 0,15 bis 4,5 %, bevorzugt im Bereich von 0,2 bis 4,0 %, besonders bevorzugt im Bereich von 0,25 bis 3,5 %, ganz besonders bevorzugt im Bereich von 0,3 bis 3,0 %, umfasst. Bevorzugt ist zusätzlich oder alternativ zudem eine erfindungsgemäße strahlungshärtende Zusammensetzung, wobei der Quotient aus dem kombinierten Stoffmengenanteil der einen oder der mehreren Hexaarylbiimidazol-Verbindungen geteilt durch den kombinierten Stoffmengenanteil der einen oder der mehreren Mercaptotetrazol-Verbindungen 1,0 oder weniger, bevorzugt 0,8 oder weniger, besonders bevorzugt 0,6 oder weniger, insbesondere 0,4 oder weniger, beträgt.

Hexaarylbiimidazol-Verbindungen und deren Einsatz als Fotoinitiator sind aus dem Stand der Technik bekannt und bspw. in der US 2017/0266081 A1 offenbart. In den Experimenten der Erfinder hat sich herauskristallisiert, dass insbesondere mit substituierten Hexaarylbiimidazol-Verbindungen besonders leistungsfähige Systeme erhalten werden können. Bevorzugt ist entsprechend eine erfindungsgemäße strahlungshärtende Zusammensetzung, wobei die eine oder die mehreren Hexaarylbiimidazol-Verbindungen ausgewählt sind aus der Gruppe bestehend aus Hexaarylbiimidazol-Verbindungen mit einem oder mehr, bevorzugt zwei oder mehr, besonders bevorzugt drei oder mehr, ganz besonders bevorzugt vier oder mehr mit Substituenten substituierten Aryl-Gruppen. Besonders bevorzugt ist dabei eine erfindungsgemäße strahlungshärtende Zusammensetzung, wobei die Substituenten ausgewählt sind aus der Gruppe bestehend aus Halogenatomen und Alkoxy-Gruppen, bevorzugt Chloratomen und Methoxy-Gruppen.

Besonders günstige Ergebnisse in den Gesamtleistungseigenschaften wurden von den Erfindern dabei insbesondere und auch durch den kombinierten Einsatz von zwei oder mehr verschiedenen Hexaarylbiimidazol-Verbindungen erreicht, wodurch sich insbesondere die Polymerisationseigenschaften der erfindungsgemäßen strahlungshärtenden Zusammensetzungen gezielt an die jeweiligen Anwendungserfordernisse anpassen lassen. Bevorzugt ist somit eine erfindungsgemäße strahlungshärtende Zusammensetzung, wobei die strahlungshärtende Zusammensetzung zwei oder mehr verschiedene Hexaarylbiimidazol-Verbindungen umfasst.

Hexaarylbiimidazol-Verbindungen können hinsichtlich der Verknüpfung der Imidazol-Ringe prinzipiell unterschiedlich ausgeführt sein. Die Erfinder haben dabei insbesondere mit solchen Verbinndungen gute Ergebnisse erzielt, in denen die Verknüpfung über eine N-N-Bindung bzw. über eine C-N-Bindung erfolgte, wobei sich für die C-N-Verknüpfung in der Gesamtzusammenschau besonders vorteilhafte Ergebnisse zeigten. Bevorzugt ist für einige Anwendungen eine erfindungsgemäße strahlungshärtende Zusammensetzung, wobei die eine oder die mehreren Hexaarylbiimidazol-Verbindungen ausgewählt sind aus der Gruppe bestehend aus Hexaaryl-1,1'-biimidazol-Verbindungen, und/oder wobei die eine oder die mehreren Hexaarylbiimidazol-Verbindungen ausgewählt sind aus der Gruppe bestehend aus Hexaarylbiimidazol-Verbindungen, in denen die Imidazol-Ringe über eine C-C-Bindung verknüpft sind. Bevorzugt ist in den meisten Fällen jedoch eine erfindungsgemäße strahlungshärtende Zusammensetzung, wobei die eine oder die mehreren Hexaarylbiimidazol-Verbindungen ausgewählt sind aus der Gruppe bestehend aus Hexaaryl-1,2'-biimidazol-Verbindungen, und/oder wobei die eine oder die mehreren Hexaarylbiimidazol-Verbindungen ausgewählt sind aus der Gruppe bestehend aus Hexaarylbiimidazol-Verbindungen, in denen die Imidazol-Ringe über eine C-N-Bindung verknüpft sind.

Im Zuge der durchgeführten Experimente war es den Erfindern möglich, besonders geeignete Hexaarylbiimidazol-Verbindungen zu identifizieren und unter den geeigneten Verbindungen darüber hinaus drei besonders leistungsstarke Vertreter dieser Verbindungsklasse zu identifizieren. Bevorzugt ist zunächst eine erfindungsgemäße strahlungshärtende Zusammensetzung, wobei die eine oder die mehreren Hexaarylbiimidazol-Verbindungen ausgewählt sind aus der Gruppe bestehend aus 2,2`-Bis(2-chlorphenyl)-4,4`,5,5`-tetraphenyl-1,2`-biimidazol (o-Cl-HABI), 2,2`-Bis(2,4-dichlorphenyl)-4,4`,5,5`-tetraphenyl-1,2`-biimidazol (2,4-Cl-HABI), 2,2`-Bis(3-chlorphenyl)-4,4`,5,5`-tetraphenyl-1,2`-biimidazol (3-Cl-HABl), 2,2`-Bis(4-chlorphenyl)-4,4`,5,5`-tetraphenyl-1,2`-biimidazol (4-Cl-HABl), 2,2`-Bis(phenyl)-4,4`-bi(2-chlorphenyl)-5,5`-biphenyl-1,2`-biimidazol, 2,2`-Bis(phenyl)-4,4`-biphenyl-5,5`-bi(2-chlorphenyl)-1,2`-biimidazol, 2,2'-Bis(phenyl)-4,4',5,5'-tetra(2-chlorphenyl)-1,2`-biimidazol 2, 2',4-Tris(2-chlorophenyl)-5-(3,4-dimethoxyphenyl)-4',5'-diphenyl-1, 2'-biimidazol und 2,2',4-Tris(2-chlorophenyl)-5-(3,4-dimethoxyphenyl)-4',5'-diphenyl-1,1'-biimidazol. Besonders bevorzugt ist zusätzlich oder alternativ auch eine erfindungsgemäße strahlungshärtende Zusammensetzung, wobei die eine oder die mehreren Hexaarylbiimidazol-Verbindungen ausgewählt sind aus der Gruppe bestehend aus 2,2'-Bis(2-chlorophenyl)-4, 4',5,5'-tetraphenyl-1,2'-biimidazol 2,2',4-Tris(2-chlorophenyl)-5-(3,4-dimethoxyphenyl)-4',5'-diphenyl-1,2'-biimidazol und 2,2',4-Tris(2-chlorophenyl)-5-(3,4-dimethoxyphenyl)-4',5'-diphenyl-1,1'-biimidazol. Besonders bevorzugt ist zusätzlich oder alternativ auch eine erfindungsgemäße strahlungshärtende Zusammensetzung, wobei die strahlungshärtende Zusammensetzung als Hexaarylbiimidazol-Verbindungen 2, 2'-Bis(2-chlorophenyl)-4,4',5,5'-tetraphenyl-1,2'-biimidazol und/oder 2,2',4-Tris(2-chlorophenyl)-5-(3, 4-dimethoxyphenyl)-4',5'-diphenyl-1,2'-biimidazol und/oder 2,2',4-Tris(2-chlorophenyl)-5-(3,4-dimethoxyphenyl)-4',5'-diphenyl-1,1'-biimidazol, bevorzugt 2, 2'-Bis(2-chlorophenyl)-4,4',5,5'-tetraphenyl-1,2'-biimidazol und 2,2',4-Tris(2-chlorophenyl)-5-(3,4-dimethoxyphenyl)-4',5'-diphenyl-1,2'-biimidazol, umfasst.

Hexaarylbiimidazol-Verbindungen wurden im Stand der Technik gemäß US 2017/0266081 A1 in Kombination mit Co-Initiatoren eingesetzt, bei denen es sich um Thiol-gruppenhaltige heterozyklische aromatische Verbindungen handelt, insbesondere Benzothiazol- und Triazol-basierte Verbindungen, nämlich mit MMT (3-Mercapto-4-methyl-4H-1,2,4-triazol) und MBT (2-Mercaptobenzothiazol).

Im Rahmen der vorliegenden Erfindung haben die Erfinder gefunden, dass die vorstehend beschriebenen Aufgaben in vorteilhafter Weise gelöst werden, wenn statt den aus dem Stand der Technik bekannten Co-Initiatoren stattdessen Mercaptotetrazol-Verbindungen eingesetzt werden. Entsprechende Mercaptotetrazol-Verbindungen sind aus anderen Anwendungsbereichen dabei grundsätzlich bekannt und von verschiedenen Herstellern kommerziell erhältlich, bspw. von der Firma Merck.

Im Zuge der eigenen Entwicklung haben die Erfinder gefunden, dass sich insbesondere durch den Einsatz von substituierten Mercaptotetrazol-Verbindungen vorteilhafte Initiatorsysteme erhalten lassen. Bevorzugt ist somit eine erfindungsgemäße strahlungshärtende Zusammensetzung, wobei die eine oder die mehreren Mercaptotetrazol-Verbindungen ausgewählt sind aus der Gruppe bestehend aus Mercaptotetrazol-Verbindungen mit zumindest einem Substituenten am Tetrazol-Ring, bevorzugt genau einem Substituenten am Tetrazol-Ring, bevorzugt ausgewählt sind aus der Gruppe bestehend aus Mercaptotetrazol-Verbindungen mit genau einem zu der Thiol-Gruppe benachbarten Substituenten. Besonders bevorzugt ist dabei eine erfindungsgemäße strahlungshärtende Zusammensetzung, wobei der Substituent ausgewählt ist aus der Gruppe bestehenden aus Substituenten mit einem +M-Effekt, und/oder wobei der Substituent ausgewählt ist aus der Gruppe bestehenden aus unsubstituierten oder substituierten Aryl-Gruppen, bevorzugt substituierten Aryl-Gruppen mit einem Substituenten in para-Stellung zum Tetrazol-Ring, wobei der Substituent bevorzugt ausgewählt ist aus der Gruppe bestehend aus Hydroxy-Gruppen und Alkoxgruppen, bevorzugt aus der Gruppe bestehend aus Hydroxy-Gruppen und Alkoxgruppen mit 1 bis 3 Kohlenstoffatomen, bevorzugt Hydroxy-Gruppen und Alkoxgruppen mit 1 oder 2 Kohlenstoffatomen.

Unter den infrage kommenden Mercaptotetrazol-Verbindungen konnten die Erfinder dabei solche identifizieren, welche sich im Zusammenspiel mit den erfindungsgemäß einzusetzenden Hexaarylbiimidazol-Verbindungen als besonders vorteilhaft erwiesen haben, insbesondere in den spezifischen Füllstoffarmen strahlungshärtenden Zusammensetzungen der vorliegenden Erfindung. Bevorzugt ist nämlich eine erfindungsgemäße strahlungshärtende Zusammensetzung, wobei die eine oder die mehreren Mercaptotetrazol-Verbindungen ausgewählt sind aus der Gruppe bestehend aus 5-Mercapto-1-phenyl-1H-tetrazol (MPHTA), 1-(4-hydroxyphenyl)-5-mercapto-1H-tetrazol (HPMTA), 1-(4-Ethoxyphenyl)-5-mercapto-1H-tetrazol (EPMATA), 1-(4-Carboxyphenyl)-5-mercapto-1H-tetrazol, 4-(5-sulfanyl-1H-1,2,3,4-tetrazol-1yl)benzonitril (STABN) und 1-[4-(5-Mercapto-1H-tetrazol-1-yl)phenyl]ethanon (MTPE). Bevorzugt ist zusätzlich oder alternativ auch eine erfindungsgemäße strahlungshärtende Zusammensetzung, wobei die eine oder die mehreren Mercaptotetrazol-Verbindungen ausgewählt sind aus der Gruppe bestehend aus 5-Mercapto-1-phenyl-1H-tetrazole (MPHTA), 1-(4-Hydroxyphenyl)-5-mercapto-1H-tetrazol (HPMTA) und 1-(4-Ethoxyphenyl)-5-mercapto-1H-tetrazol (EPMTA).

In Anlehnung an die vorstehenden Ausführungen zum kombinierten Einsatz von zwei oder mehr verschiedenen Hexaarylbiimidazol-Verbindungen schlagen die Erfinder vor, dass auch unterschiedliche Mercaptotetrazol-Verbindungen eingesetzt werden können, um insbesondere das Polymerisationsverhalten zielgenau an die jeweiligen Anwendungserfordernisse anzupassen. Bevorzugt ist entsprechend eine erfindungsgemäße strahlungshärtende Zusammensetzung, wobei die strahlungshärtende Zusammensetzung zwei oder mehr verschiedene Mercaptotetrazol-Verbindungen umfasst.

Bevorzugt ist zusätzlich oder alternativ auch eine erfindungsgemäße strahlungshärtende Zusammensetzung, wobei die strahlungshärtende Zusammensetzung als Hexaarylbiimidazol-Verbindung 2, 2'-Bis(2-chlorophenyl)-4, 4', 5, 5'-tetraphenyl-1, 2'-biimidazol und als Mercaptotetrazol-Verbindung 5-Mercapto-1-phenyl-1H-tetrazole (MPHTA) umfasst.

Es kann als Vorteil der erfindungsgemäßen strahlungshärtenden Zusammensetzungen angesehen werden, dass diese hinsichtlich der Anwesenheit von üblichen Additiven sehr kompatibel sind, so dass durch den Einsatz solcher Additive ein vorteilhaftes und an den jeweiligen Anwendungsfall abgestimmtes Eigenschaftsprofil eingestellt werden kann. Bevorzugt ist somit in vielen Fällen eine erfindungsgemäße strahlungshärtende Zusammensetzung, wobei die strahlungshärtende Zusammensetzung zusätzlich ein oder mehrere Additive umfasst, bevorzugt mit einem kombinierten Massenanteil im Bereich von 0,01 bis 10 %, vorzugsweise im Bereich von 0,05 bis 5 %, besonders bevorzugt im Bereich von 0,1 bis 2 %, wobei die Additive bevorzugt ausgewählt sind aus der Gruppe bestehend aus Farbstoffen, Fließverbesserern, Thixotropiemitteln, Verdickern, Stabilisatoren und UV-Schutzmitteln.

Nachfolgend werden strahlungshärtende Zusammensetzungen offenbart, die nach Einschätzung der Erfinder besonders bevorzugt sind und die in ganz besonders bevorzugten Ausführungsformen mit einem, zwei oder mehreren der vorstehend als bevorzugt bezeichneten Merkmalen kombiniert werden.

Ein erstes bevorzugtes Ausführungsbeispiel ist eine strahlungshärtende Zusammensetzung eine erfindungsgemäße strahlungshärtende Zusammensetzung, umfassend bezogen auf die Gesamtmasse der strahlungshärtenden Zusammensetzung:
i) ein oder mehrere radikalisch polymerisierbare Monomere in einem kombinierten Massenanteil von 70 % oder mehr,
ii) einen oder mehrere Füllstoffe in einem kombinierten Massenanteil im Bereich von 0,05 bis 29 %,
iii) eine oder mehrere Hexaarylbiimidazol-Verbindungen in einem kombinierten Massenanteil im Bereich von 0,1 bis 5 %, und
iiiv) eine oder mehrere Mercaptotetrazol-Verbindungen in einem kombinierten Massenanteil im Bereich von 0,1 bis 5 %,
wobei der kombinierte Massenanteil an Füllstoffen in der strahlungshärtenden Zusammensetzung weniger als 30 % beträgt.

Ein zweites bevorzugtes Ausführungsbeispiel ist eine strahlungshärtende Zusammensetzung eine erfindungsgemäße strahlungshärtende Zusammensetzung, umfassend bezogen auf die Gesamtmasse der strahlungshärtenden Zusammensetzung:
i) ein oder mehrere radikalisch polymerisierbare Monomere in einem kombinierten Massenanteil von 70 % oder mehr, wobei das eine oder die mehreren radikalisch polymerisierbaren Monomere ausgewählt sind aus der Gruppe bestehend aus (Meth)acrylsäure, (Meth)acrylaten, (Meth)acrylamiden und anderen Vinyl-Verbindungen,
ii) einen oder mehrere Füllstoffe in einem kombinierten Massenanteil im Bereich von 0,5 bis 22,5 %,
iii) eine oder mehrere Hexaarylbiimidazol-Verbindungen in einem kombinierten Massenanteil im Bereich von 0,1 bis 5 %, wobei die eine oder die mehreren Hexaarylbiimidazol-Verbindungen ausgewählt sind aus der Gruppe bestehend aus 2,2'-Bis(2-chlorophenyl)-4, 4',5,5'-tetraphenyl-1,2'-biimidazol 2,2',4-Tris(2-chlorophenyl)-5-(3,4-dimethoxyphenyl)-4',5'-diphenyl-1,2'-biimidazol und 2,2',4-Tris(2-chlorophenyl)-5-(3,4-dimethoxyphenyl)-4',5'-diphenyl-1,1'-biimidazol, und
iiiv) eine oder mehrere Mercaptotetrazol-Verbindungen in einem kombinierten Massenanteil im Bereich von 0,1 bis 5 %, wobei die eine oder die mehreren Mercaptotetrazol-Verbindungen ausgewählt sind aus der Gruppe bestehend aus 5-Mercapto-1-phenyl-1H-tetrazole (MPHTA), 1-(4-Hydroxyphenyl)-5-mercapto-1H-tetrazol (HPMTA) und 1-(4-Ethoxyphenyl)-5-mercapto-1H-tetrazol (EPMTA),
wobei der kombinierte Massenanteil an Füllstoffen in der strahlungshärtenden Zusammensetzung weniger als 25 % beträgt.

Ein drittes bevorzugtes Ausführungsbeispiel ist eine strahlungshärtende Zusammensetzung eine erfindungsgemäße strahlungshärtende Zusammensetzung, umfassend bezogen auf die Gesamtmasse der strahlungshärtenden Zusammensetzung:
i) ein oder mehrere radikalisch polymerisierbare Monomere in einem kombinierten Massenanteil von 75 % oder mehr, wobei das eine oder die mehreren radikalisch polymerisierbaren Monomere ausgewählt sind aus der Gruppe bestehend aus monofunktionellen (Meth)acrylaten und polyfunktionellen (Meth)acrylaten,
ii) einen oder mehrere Füllstoffe in einem kombinierten Massenanteil im Bereich von 1 bis 20 %,
iii) eine oder mehrere Hexaarylbiimidazol-Verbindungen in einem kombinierten Massenanteil im Bereich von 0,2 bis 4 %, wobei die eine oder die mehreren Hexaarylbiimidazol-Verbindungen ausgewählt sind aus der Gruppe bestehend aus 2,2'-Bis(2-chlorophenyl)-4, 4',5,5'-tetraphenyl-1,2'-biimidazol 2,2',4-Tris(2-chlorophenyl)-5-(3,4-dimethoxyphenyl)-4',5'-diphenyl-1,2'-biimidazol und 2,2',4-Tris(2-chlorophenyl)-5-(3,4-dimethoxyphenyl)-4',5'-diphenyl-1,1'-biimidazol, und
iiiv) eine oder mehrere Mercaptotetrazol-Verbindungen in einem kombinierten Massenanteil im Bereich von 0,2 bis 4 %, wobei die eine oder die mehreren Mercaptotetrazol-Verbindungen ausgewählt sind aus der Gruppe bestehend aus 5-Mercapto-1-phenyl-1H-tetrazole (MPHTA), 1-(4-Hydroxyphenyl)-5-mercapto-1H-tetrazol (HPMTA) und 1-(4-Ethoxyphenyl)-5-mercapto-1H-tetrazol (EPMTA),

wobei der kombinierte Massenanteil an Füllstoffen in der strahlungshärtenden Zusammensetzung weniger als 25 % beträgt,
wobei die strahlungshärtende Zusammensetzung bei 23 °C eine dynamische Viskosität im Bereich von 0,001 bis 10 Pa s aufweist.

Die Erfindung betrifft auch ein dentales Bauteil, hergestellt oder herstellbar in einem DLP-Verfahren oder SLA-Verfahren durch Strahlungshärtung einer erfindungsgemäßen strahlungshärtenden Zusammensetzung, insbesondere einer bevorzugten erfindungsgemäßen strahlungshärtenden Zusammensetzung.

Bevorzugt ist dabei im Lichte der vorstehenden Ausführungen ein erfindungsgemäßes dentales Bauteil, wobei das dentale Bauteil hergestellt oder herstellbar ist in einem DLP-Verfahren oder SLA-Verfahren durch Strahlungshärtung einer strahlungshärtenden Zusammensetzung mit elektromagnetischer Strahlung im Wellenlängenbereich von 200 und 420 nm, bevorzugt im Wellenlängenbereich von 250 bis 415 nm, besonders bevorzugt im Wellenlängenbereich von 300 bis 410 nm, auszuhärten.

Die Erfindung betrifft auch die Verwendung eines Initiatorsystems in einer strahlungshärtenden Zusammensetzung, zur Verbesserung der mechanischen Eigenschaften der im DLP-Verfahren oder SLA-Verfahren daraus herstellbaren dentalen Bauteile zur Verringerung gesundheitlicher Bedenken, wobei die strahlungshärtenden Zusammensetzung bezogen auf die Gesamtmasse der strahlungshärtenden Zusammensetzung umfasst:
i) ein oder mehrere radikalisch polymerisierbare Monomere in einem kombinierten Massenanteil von 60 % oder mehr,
   wobei der kombinierte Massenanteil an Füllstoffen in der strahlungshärtenden Zusammensetzung weniger als 30 % beträgt,
   wobei das Initiatorsystem bezogen auf die Gesamtmasse der strahlungshärtenden Zusammensetzung umfasst:
      x) eine oder mehrere Hexaarylbiimidazol-Verbindungen in einem kombinierten Massenanteil im Bereich von 0,1 bis 5 %, und
   y) eine oder mehrere Mercaptotetrazol-Verbindungen in einem kombinierten Massenanteil im Bereich von 0,1 bis 5 %.

Nachfolgend werden die Erfindung und bevorzugte Ausführungsformen der Erfindung unter Bezugnahme auf Experimente näher erläutert und beschrieben.

### A. Herstellung der strahlungshärtenden Zusammensetzungen:

Die Herstellung der strahlungshärtenden Zusammensetzungen erfolgte in branchenüblicher Weise durch Vermischen der Komponenten.

Der Einfluss der Initiatorsysteme wurde dabei in ausgehend von einem nicht erfindungsgemäßen Modellsystem M1 untersucht, welches nach der Erfahrung der Erfinder hinsichtlich seiner Zusammensetzung ausgezeichnet geeignet ist, als Ausgangspunkt für eine Studie zur Wirkung der Initiatorsysteme zu fungieren. Das Modellsystem M1 hatte die in Tabelle 1 angegebene Zusammensetzung.

**Tabelle 1 - Zusammensetzung Modellsystem M1**

| **Handelsname** | **Chemische Bezeichnung** | **Massenanteil / %** |
|---|---|---|
| Sartomer SR348L | Ethoxyliertes 2 Bisphenol A Dimethacrylat | 4,50 |
| Sartomer SR789 | Tricyclodecanmethanol Acrylat | 28,40 |
| Genomer 4297 | Urethandimethacrylat | 46,00 |
| Genomer 7151 | Carboxy-funktionalisiertes Polyester Acrylat | 6,90 |
| Omnirad 819 | Phenylbis (2,4,6-trimethylbenzoyl) phosphinoxid | 1,00 |
| Aerosil ox. 50 sil. | Gefälltes Siliziumdioxid | 9,00 |
| Zirkonsil 520 | Mischung Zirkonium- und Siliziumdioxid | 4,00 |
| Eusolex 4360 (UV09) | Butyliertes Hydroxytoluol | 0,20 |
| Lumilux Blau | Terephthalsäure-ester | 0,006 |

Aus gehend von dem Modellsystem wurden strahlungshärtenden Zusammensetzungen hergestellt, indem das Phenylbis (2,4,6-trimethylbenzoyl) phosphinoxid gegen ein anderes Initiatorsystem ausgetauscht wurde. Sofern hierbei ein größere Gesamtmassenanteil des Initiatorsystems eingestellt wurde, reduzierte sich der relative Massenanteil der weiteren Komponenten entsprechend. Die Menge des Co-Initiators (MTA, MPHTA, HPMTA und EPMTA) wurde dabei im Hinblick auf die Stoffmenge des zugebenen Sensibilisators (HABI) berechnet und zugegeben, wobei das Stoffmengenverhältnis teilweise variiert wurde.

Es wurden die in Tabelle 2 zusammengefassten strahlungshärtenden Zusammensetzungen Z1 bis Z17 hergestellt. Hierbei ist die Zusammensetzung Z1 nicht erfindungsgemäß.

**Tabelle 2 - Zusammensetzung der strahlungshärtenden Zusammensetzungen Z1 bis Z17 (alle Angaben in Massenanteilen)**

| Nr. | HABI-1 | HABI-2 | HABI-3 | MTA | MPHTA | HPMTA | EPMTA |
|---|---|---|---|---|---|---|---|
| Z1 | 1,0 | | | 0,46 | | | |
| Z2 | 1,0 | | | | 0,81 | | |
| Z3 | 1,0 | | | | | 0,88 | |
| Z4 | 1,0 | | | | | | 1,01 |
| Z5 | 0,5 | | | | | | 0,5 |
| Z6 | 1,5 | | | | | | 1,52 |
| Z7 | 2,0 | | | | 1,62 | | |
| Z8 | 1,0 | | | | 0,27 | | |
| Z9 | 1,0 | | | | 0,54 | | |
| Z10 | 1,0 | | | | 1,35 | | |
| Z11 | 1,0 | | | | 0,71 | | |
| Z12 | 0,5 | 0,5 | | | 0,76 | | |
| Z13 | 0,35 | 0,45 | | | 0,61 | | |
| Z14 | 0,18 | 0,42 | | | 0,44 | | |
| Z15 | | | 1,0 | | 0,71 | | |
| Z16 | | | 0,5 | | 0,36 | | |
| Z17 | 0,5 | | 0,5 | | 0,71 | | |

Die vorstehend verwendeten Abkürzungen entsprechen dabei den in Tabelle 3 dargestellten Verbindungen.

**Tabelle 3 - Eingesetzte Verbindungen**

| Abk. | Formel | Struktur | Anmerkung |
|---|---|---|---|
| HABI-1 | 2,2'-Bis(2-chlorophenyl)-4,4',5,5'-tetraphenyl-1,2'-biimidazol | 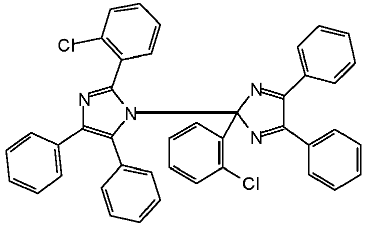 | |
| HABI-2 | 2,2',4-Tris(2-chlorophenyl)-5-(3,4-dimethoxyphenyl)-4',5'-diphenyl-1,2'-biimidazol | 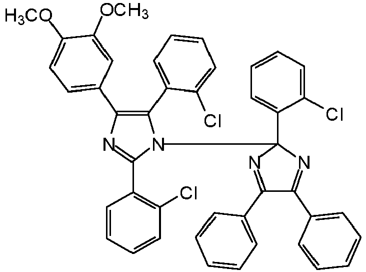 | |
| HABI-3 | 2,2',4-Tris(2-chlorophenyl)-5-(3,4-dimethoxyphenyl)-4',5'-diphenyl-1,1'-biimidazol | 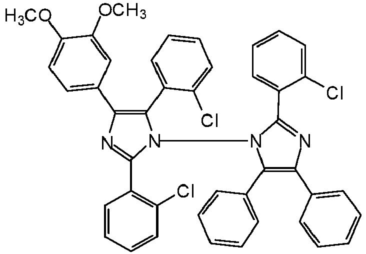 | |
| MTA | 3-Mercapto-1,2,4-triazole | 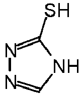 | |
| MPHTA | 5-Mercapto-1-phenyl-1H-tetrazole | 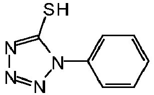 | +M-Effekt schwach |
| HPMTA | 1-(4-Hydroxyphenyl)-5-mercapto-1H-tetrazol | 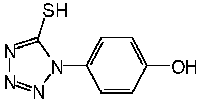 | +M-Effekt ausgeprägt |
| EPMTA | 1-(4-Ethoxyphenyl)-5-mercapto-1H-tetrazol | 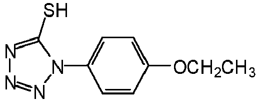 | +M-Effekt |

### B. Reaktivitätsexperimente:

An den hergestellten Proben wurden zunächst die Reaktivität und die Aushärtekinetik untersucht. Hierfür wurden die strahlungshärtenden Zusammensetzungen mit einer unterschiedlichen Strahlungsdosis beaufschlagt und die resultierende mittlere Schichtdicke bestimmt.

Der DLP-Drucker (cara^{®} Print 4.0 pro) wurde mindestens 5 Minuten vor Nutzung aktiviert. Die Messungen wurden bei einer Umgebungstemperatur zwischen 21 °C und 25 °C durchgeführt, wobei die Temperatur der zu messenden Zusammensetzungen 21 °C nicht unterschreiten und 25 °C nicht überschreiten durfte. Das Vat, d.h. der Reservoirbehälter für das zu verdruckende Photopolymer, in den ein Belichtungsfenster aus beschichtetem Glas eingebracht ist, wurde in den Drucker eingeführt und Hostaphanfolie (3 cm x 6 cm) mittig auf der Vat-Folie platziert. Initial wurde die Belichtungsintensität mit Hilfe eines geeigneten Radiometers (Opsytec Dr. Gröbel RM 12) ermittelt, wobei sowohl mit als auch ohne Hostaphanfolie gemessen wurde. Anschließend wurde mit einer Einwegpipette aus Kunststoff ein erbsengroßer Tropfen der Zusammensetzung auf die Hostaphanfolie aufgetragen und mit einer definierten Belichtungsdosis bestrahlt. Sobald der Belichtungsprozess beendet war, wurde die Hostaphanfolie mit dem teilweise ausgehärteten Material aus dem Vat entnommen und unter eine, bezüglich der Hostaphanfolie, genullte digitale Messuhr (Sylvac S_Dial Work Nano) gelegt. Nach einer Minute wurde der Messwert in Form der erzeugten Schichtdicke abgelesen und dokumentiert. Je Belichtungsdosis wurden vier Messungen durchgeführt. Die mittlere Schichtdicke errechnet sich aus dem arithmetischen Mittel der bestimmten Schichtdicken.

Die erhaltenen Ergebnisse sind in der nachfolgenden Tabelle 4 zusammengestellt.

**Tabelle 4 - Ergebnisse der Reaktivitätsexperimente**

| **Nr.** | **Dosis / (mJ/cm²)** | **In(Dosis)** | **Mittlere Schichtdicke / µm** |
|---|---|---|---|
| *M1* | 5,570 | 1,7174 | 49,9 |
| | 7,798 | 2,0539 | 112,6 |
| | 10,026 | 2,3052 | 157,1 |
| | 12,254 | 2,5059 | 191,5 |
| | 15,596 | 2,7470 | 231,3 |
| | | | |
| *Z1* | 15,465 | 2,7386 | 41,2 |
| | 16,496 | 2,8031 | 100,0 |
| | 17,527 | 2,8637 | 159,1 |
| | 20,620 | 3,0263 | 265,6 |
| | 30,930 | 3,4317 | 502,1 |
| | | | |
| *Z2* | 16,496 | 2,8031 | 60,8 |
| | 17,527 | 2,8637 | 102,3 |
| | 20,620 | 3,0263 | 204,2 |
| | 23,713 | 3,1660 | 276,0 |
| | 30,930 | 3,4317 | 423,3 |
| | | | |
| *Z3* | 15,465 | 2,7386 | 72,3 |
| | 16,496 | 2,8031 | 123,6 |
| | 20,620 | 3,0263 | 262,7 |
| | 25,775 | 3,2494 | 386,7 |
| | 30,930 | 3,4317 | 475,6 |
| | | | |
| *Z4* | 16,496 | 2,8031 | 53,4 |
| | 17,527 | 2,8637 | 104,9 |
| | 21,651 | 3,0751 | 232,8 |
| | 25,775 | 3,2494 | 324,0 |
| | 30,930 | 3,4317 | 420,0 |
| | | | |
| *Z5* | 35,904 | 3,5808 | 51,5 |
| | 36,960 | 3,6098 | 91,6 |
| | 39,072 | 3,6654 | 157,2 |
| | 42,240 | 3,7434 | 241,0 |
| | 52,800 | 3,9665 | 409,0 |
| | | | |
| *Z6* | 12,372 | 2,5154 | 68,8 |
| | 13,403 | 2,5955 | 120,4 |
| | 14,434 | 2,6696 | 163,9 |
| | 16,496 | 2,8031 | 216,3 |
| | 20,620 | 3,0263 | 314,8 |
| | | | |
| *Z7* | 12,396 | 2,5174 | 87,3 |
| | 13,429 | 2,5974 | 116,8 |
| | 14,462 | 2,6715 | 145,7 |
| | 16,528 | 2,8051 | 188,9 |
| | 20,660 | 3,0282 | 269,1 |
| | | | |
| *Z8* | 21,500 | 3,0681 | 47,0 |
| | 22,575 | 3,1168 | 89,4 |
| | 24,725 | 3,2078 | 151,8 |
| | 27,950 | 3,3304 | 220,7 |
| | 32,250 | 3,4735 | 295,0 |
| | | | |
| *Z9* | 19,350 | 2,9627 | 61,1 |
| | 21,500 | 3,0681 | 136,0 |
| | 23,650 | 3,1634 | 192,3 |
| | 27,950 | 3,3304 | 280,8 |
| | 32,250 | 3,4735 | 356,2 |
| | | | |
| *Z10* | 16,125 | 2,7804 | 53,8 |
| | 17,200 | 2,8449 | 101,2 |
| | 19,350 | 2,9627 | 179,5 |
| | 23,650 | 3,1634 | 287,6 |
| | 26,875 | 3,2912 | 352,9 |
| | | | |
| *Z11* | 5,165 | 1,6419 | 69,7 |
| | 7,231 | 1,9784 | 95,4 |
| | 10,330 | 2,3351 | 124,4 |
| | 14,462 | 2,6715 | 152,5 |
| | 20,660 | 3,0282 | 187,2 |
| | | | |
| *Z12* | 5,285 | 1,6649 | 49,4 |
| | 7,399 | 2,0013 | 106,7 |
| | 9,513 | 2,2527 | 151,5 |
| | 13,741 | 2,6204 | 218,5 |
| | 21,140 | 3,0512 | 304,6 |
| | | | |
| Z13 | 6,342 | 1,8472 | 60,2 |
| | 7,399 | 2,0013 | 90,6 |
| | 9,513 | 2,2527 | 140,7 |
| | 13,741 | 2,6204 | 212,1 |
| | 21,140 | 3,0512 | 305,9 |
| | | | |
| Z14 | 7,399 | 2,0013 | 63,2 |
| | 9,513 | 2,2527 | 117,0 |
| | 11,627 | 2,4533 | 159,0 |
| | 14,798 | 2,6945 | 210,6 |
| | 21,140 | 3,0512 | 292,5 |
| | | | |
| Z15 | 7,658 | 2,0358 | 54,1 |
| | 10,940 | 2,3924 | 64,6 |
| | 21,880 | 3,0856 | 101,6 |
| | 32,820 | 3,4910 | 115,5 |
| | 54,700 | 4,0019 | 164,9 |
| | | | |
| Z16 | 25,979 | 2,9957 | 43,6 |
| | 38,969 | 3,4012 | 78,0 |
| | 51,959 | 3,6889 | 109,6 |
| | 64,948 | 3,9120 | 132,5 |
| | 103,918 | 4,3820 | 206,0 |
| | | | |
| Z17 | 6,564 | 1,8816 | 43,8 |
| | 10,940 | 2,3924 | 74,9 |
| | 21,880 | 3,0856 | 116,8 |
| | 32,820 | 3,4910 | 146,5 |
| | 65,640 | 4,1842 | 212,3 |

Aus diesen Messwerten können aus der linearen Regression in der Auftragung von Schichtdicke gegen In(Dosis) die minimalen Belichtungsdosis Emin (als Schnittpunkt der lineare Regression mit x-Achse) und der Zusammenhang von erzeugter Schichtdicke und Lichtdosis als dp (als Steigung der linearen Regression) abgeleitet werden. Die Ergebnisse sind in der Tabelle 5 zusammengestellt.

**Tabelle 5 - minimalen Belichtungsdosis Emin und Steigung der Regression dp**

| Nr. | *E*ₘᵢₙ / (mJ/cm²) | *dp* |
|---|---|---|
| *M1* | 1,42 | 250,80 |
| *Z1* | 14,07 | 649,70 |
| *Z2* | 14,66 | 572,38 |
| *Z3* | 13,39 | 580,41 |
| *Z4* | 14,73 | 575,68 |
| *Z5* | 33,27 | 911,35 |
| *Z6* | 10,43 | 468,95 |
| *Z7* | 9,65 | 353,61 |
| *Z8* | 19,54 | 602,93 |
| *Z9* | 17,10 | 568,92 |
| *Z10* | 14,48 | 580,30 |
| *Z11* | 2,31 | 84,33 |
| *Z12* | 4,12 | 183,94 |
| *Z13* | 4,75 | 203,11 |
| *Z14* | 5,57 | 217,70 |
| *Z15* | 3,18 | 54,308 |
| *Z16* | 14,82 | 116,28 |
| *Z17* | 3,86 | 71,781 |

Aus den vorstehenden Daten ist ersichtlich, dass sich mit erfindungsgemäßen strahlungshärtenden Zusammensetzungen ausgezeichnete Ergebnisse erzielen lassen, wobei insbesondere die Härtungskinetik gezielt über einen breiten Bereich hinweg an die Anwendungserfordernisse angepasst werden kann.

Hierbei lassen sich insbesondere auch solche Werte für Eₘᵢₙ und dp erzielen, welche mit denen des etablierten Phenylbis (2,4,6-trimethylbenzoyl) phosphinoxid vergleichbar sind. Insbesondere durch die Kombination von zwei Hexaarylbiimidazol-Verbindungen in Z12, Z13 und Z14 werden dabei ausgezeichnete Werte erhalten, die hinsichtlich beider Kenngrößen sehr nah am Optimum liegen, welches jeweils nicht zu hoch, aber gleichzeitig auch nicht zu niedrig liegen soll, um beispielsweise eine vorzeitige, ungewollte Aushärtung zu verhindern. Durch die Kombination von zwei Hexaarylbiimidazol-Verbindungen ist es in den Proben Z13 und Z14 insbesondere möglich, bei vorteilhaften Aushärtungseigenschaften den Gesamtgehalt zu reduzieren, was nicht nur unter herstellungstechnischen Aspekten bevorzugt ist, sondern auch mit Blick auf die Farbeigenschaften der Zusammensetzungen Vorteile liefert.

### C. Prüfung der mechanischen Eigenschaften:

Über die vorstehenden Experimente hinaus wurden für ausgewählte strahlungshärtende Zusammensetzungen die mechanischen Eigenschaften untersucht. Hierfür wurden die strahlungshärtenden Zusammensetzungen ausgehärtet (DLP-Drucker: cara^{®} Print 4.0 pro; Nachbelichtung: Zweimal fünf Minuten mit HiLite^{®} power 3D) und die Biegefestigkeit, der E-Modul, die Brucharbeit und die Bruchzähigkeit wie folgt bestimmt:
Die Herstellung der Biegestäbchen sowie die Bestimmung der Biegefestigkeit und des Elastizitätsmoduls (E-Modul) erfolgt gemäß DIN EN ISO10477:2020 (7.5).

Die Herstellung der Probekörper sowie die Bestimmung der Brucharbeit und der Bruchzähigkeit erfolgt gemäß DIN EN ISO 20795-2:2013 (8.4).

Die Ergebnisse sind in Tabelle 6 zusammengestellt.

**Tabelle 6 - mechanische Eigenschaften der Polymerisate**

| Nr. | Biegefestigkeit / MPa | E-Modul / MPa | Brucharbeit / (J/m²) | Bruchzähigkeit / (MPa m^{1/2}) |
|---|---|---|---|---|
| *M1* | 110,4 | 2971 | 67,23 | 0,92 |
| *Z1* | 127,2 | 3289 | 63,18 | 0,90 |
| *Z2* | 129,4 | 3537 | 79,51 | 0,96 |
| *Z7* | 125,3 | 3528 | 80,45 | 0,99 |
| *Z12* | 128,0 | 3528 | 65,98 | 0,91 |

Aus den vorstehenden Daten ist ersichtlich, dass mit erfindungsgemäßen strahlungshärtenden Zusammensetzungen ausgezeichnete mechanische Eigenschaften erzielt werden können, die für die unmittelbar vergleichbaren Proben M1, Z1 und Z2 eindrucksvoll belegen, dass die erfindungsgemäßen strahlungshärtenden Zusammensetzungen den aus dem Stand der Technik bekannten Systemen deutlich überlegen sind. Die Messungen zu Z7 und Z12 bestätigen, dass sich die vorteilhaften Eigenschaftsprofile auch über einen breiten Zusammensetzungsbereich erhalten lassen.

## Patentansprüche

1. Strahlungshärtende Zusammensetzung für die Herstellung dentaler Bauteile im DLP-Verfahren oder SLA-Verfahren, umfassend bezogen auf die Gesamtmasse der strahlungshärtenden Zusammensetzung:
i) ein oder mehrere radikalisch polymerisierbare Monomere in einem kombinierten Massenanteil von 60 % oder mehr,
ii) eine oder mehrere Hexaarylbiimidazol-Verbindungen in einem kombinierten Massenanteil im Bereich von 0,1 bis 5 %, und
iii) eine oder mehrere Mercaptotetrazol-Verbindungen in einem kombinierten Massenanteil im Bereich von 0,1 bis 5 %,
wobei der kombinierte Massenanteil an Füllstoffen in der strahlungshärtenden Zusammensetzung weniger als 30 % beträgt.

2. Strahlungshärtende Zusammensetzung nach Anspruch 1, wobei die strahlungshärtende Zusammensetzung bei 23 °C eine dynamische Viskosität im Bereich von 0,001 bis 10 Pa s aufweist.

3. Strahlungshärtende Zusammensetzung nach einem der Ansprüche 1 oder 2, wobei die strahlungshärtende Zusammensetzung das eine oder die mehreren radikalisch polymerisierbaren Monomere in einem kombinierten Massenanteil von 65 % oder mehr umfasst.

4. Strahlungshärtende Zusammensetzung nach einem der Ansprüche 1 bis 3, wobei der kombinierte Massenanteil an Füllstoffen in der strahlungshärtenden Zusammensetzung 25 % oder weniger beträgt.

5. Strahlungshärtende Zusammensetzung nach einem der Ansprüche 1 bis 4, wobei die strahlungshärtende Zusammensetzung zusätzlich umfasst:
iv) einen oder mehrere Füllstoffe in einem kombinierten Massenanteil im Bereich von 0,05 bis 29 %.

6. Strahlungshärtende Zusammensetzung nach einem der Ansprüche 1 bis 5, wobei der Quotient aus dem kombinierten Stoffmengenanteil der einen oder der mehreren Hexaarylbiimidazol-Verbindungen geteilt durch den kombinierten Stoffmengenanteil der einen oder der mehreren Mercaptotetrazol-Verbindungen 1,0 oder weniger beträgt.

7. Strahlungshärtende Zusammensetzung nach einem der Ansprüche 1 bis 6, wobei die eine oder die mehreren Hexaarylbiimidazol-Verbindungen ausgewählt sind aus der Gruppe bestehend aus Hexaaryl-1,2'-biimidazol-Verbindungen.

8. Strahlungshärtende Zusammensetzung nach einem der Ansprüche 1 bis 7, wobei die strahlungshärtende Zusammensetzung zwei oder mehr verschiedene Hexaarylbiimidazol-Verbindungen umfasst.

9. Strahlungshärtende Zusammensetzung nach einem der Ansprüche 1 bis 8, wobei die eine oder die mehreren Mercaptotetrazol-Verbindungen ausgewählt sind aus der Gruppe bestehend aus Mercaptotetrazol-Verbindungen mit zumindest einem Substituenten am Tetrazol-Ring.

10. Strahlungshärtende Zusammensetzung nach Anspruch 9, wobei der Substituent ausgewählt ist aus der Gruppe bestehenden aus Substituenten mit einem +M-Effekt.

11. Strahlungshärtende Zusammensetzung nach einem der Ansprüche 9 oder 10, wobei die eine oder die mehreren Mercaptotetrazol-Verbindungen ausgewählt sind aus der Gruppe bestehend aus 5-Mercapto-1-phenyl-1H-tetrazol (MPHTA), 1-(4-hydroxyphenyl)-5-mercapto-1H-tetrazol (HPMTA), 1-(4-Ethoxyphenyl)-5-mercapto-1H-tetrazol (EPMATA), 1-(4-Carboxyphenyl)-5-mercapto-1H-tetrazol, 4-(5-sulfanyl-1H-1,2,3,4-tetrazol-1yl)benzonitril (STABN) und 1-[4-(5-Mercapto-1H-tetrazol-1-yl)phenyl]ethanon (MTPE).

12. Strahlungshärtende Zusammensetzung nach einem der Ansprüche 9 bis 11, wobei die Mercaptotetrazol-Verbindung 1-(4-hydroxyphenyl)-5-mercapto-1H-tetrazol (HPMTA) ist.

13. Strahlungshärtende Zusammensetzung nach einem der Ansprüche 9 bis 12, wobei die strahlungshärtende Zusammensetzung als Hexaarylbiimidazol-Verbindung 2, 2'-Bis(2-chlorophenyl)-4, 4', 5, 5'-tetraphenyl-1,2'-biimidazol und als Mercaptotetrazol-Verbindung 5-Mercapto-1-phenyl-1H-tetrazole (MPHTA) umfasst.

14. Dentales Bauteil, hergestellt oder herstellbar in einem DLP-Verfahren oder SLA-Verfahren durch Strahlungshärtung einer strahlungshärtenden Zusammensetzung nach einem der Ansprüche 1 bis 13.

15. Verwendung eines Initiatorsystems in einer strahlungshärtenden Zusammensetzung, zur Verbesserung der mechanischen Eigenschaften der im DLP-Verfahren oder SLA-Verfahren daraus herstellbaren dentalen Bauteile und zur Verringerung gesundheitlicher Bedenken, wobei die strahlungshärtenden Zusammensetzung bezogen auf die Gesamtmasse der strahlungshärtenden Zusammensetzung umfasst:
i) ein oder mehrere radikalisch polymerisierbare Monomere in einem kombinierten Massenanteil von 60 % oder mehr,
wobei der kombinierte Massenanteil an Füllstoffen in der strahlungshärtenden Zusammensetzung weniger als 30 % beträgt,
wobei das Initiatorsystem bezogen auf die Gesamtmasse der strahlungshärtenden Zusammensetzung umfasst:
x) eine oder mehrere Hexaarylbiimidazol-Verbindungen in einem kombinierten Massenanteil im Bereich von 0,1 bis 5 %, und
y) eine oder mehrere Mercaptotetrazol-Verbindungen in einem kombinierten Massenanteil im Bereich von 0,1 bis 5 %.
